# EUROPÄISCHE PATENTSCHRIFT

(11) **EP 3 034 098 B1**
(45) Veröffentlichungstag und Bekanntmachung des Hinweises auf die Patenterteilung: **14.02.2018**
(21) Anmeldenummer: 14198623.2
(22) Anmeldetag: 17.12.2014
(51) Int. Cl.: A61L 2/18, A61L 2/235, E05B 1/00, A01N 25/34

(54) **Verwendung einer perforierten elastischen Folie als Frontwand eines fluidabgebenden Kissens**
Use of a perforated elastic film as a front wall of a cushion that dispenses fluid
Utilisation d'une feuille élastique perforée en tant que face avant d'un coussin délivrant un fluide

(43) Veröffentlichungstag der Anmeldung: 22.06.2016
(73) Patentinhaber: SURFACESKINS LIMITED, Leeds, LS2 9JT (GB)
(72) Erfinder: Sollmann, Henner, 48599 Gronau (DE); Brüggemann, Andreas, 48691 Vreden (DE)
(74) Vertreter: White, Nicholas John

(56) Entgegenhaltungen:
- WO-A1-03/000106
- WO-A1-03/028456
- WO-A2-2013/167746
- US-B1- 6 726 386

## Beschreibung

Die vorliegende Erfindung betrifft ein Fluidabgebendes Kissen mit einer fluiddichten Rückwand aus Kunststoff, einer Frontwand aus einer perforierten elastischen Folie, welche durch eine Heißsiegelnaht mit der Rückwand verbunden ist, und mit einem fluidaufnehmenden Kern zwischen der Frontwand und der Rückwand.

Die vorliegende Erfindung bezieht sich insbesondere auf fluidabgebende Kissen, welche ein Fluid in Form einer desinfizierenden Flüssigkeit oder in Form eines desinfizierenden Gels enthalten, wobei die Flüssigkeit bzw. das Gel über einen längeren Zeitraum über die Frontwand abgegeben werden kann.

Um die Übertragung von Bakterien, Krankheitserregern oder dergleichen zu vermeiden, kann ein solches fluidabgebendes Kissen auf Schwenktüren, Türöffnern oder anderen Einrichtungen wie Schaltern angeordnet werden, wobei das auch als Pad zu bezeichnende fluidabgebende Kissen die von einem Benutzer zu berührende Kontaktfläche bildet.

Durch das fluidabgebende Kissen kann also eine solche Kontaktfläche frei von Keimen, Bakterien oder Krankheitserregern gehalten werden, selbst wenn die Kontaktfläche von einer Vielzahl von Benutzern berührt wird. Darüber hinaus ergibt sich auch der Vorteil, dass die von einem Benutzer auf die Kontaktfläche geführten Körperteile wie insbesondere Finger in einem gewissen Maße auch mit dem Desinfektionsmittel benetzt werden können, wodurch auch diese Körperstellen in einem gewissen Maße gereinigt werden können.

Der Einsatz eines solchen fluidabgebenden Kissens mit einem desinfizierenden Fluid ist also gerade dort von Vorteil, wo die erhöhte Gefahr einer Krankheitsübertragung besteht. Neben sämtlichen Bereichen mit einem erhöhten Personalaufkommen, wie beispielsweise Ladengeschäfte, Restaurants und Bürogebäuden ist der Einsatz des fluidabgebenden Kissens beispielsweise auch im Bereich von Krankenhäusern oder auch auf Kreuzfahrschiffen von Vorteil, um eine Ausbreitung von Krankheiten zu vermeiden.

Um gute Gebrauchseigenschaften des fluidabgebenden Kissens zu erreichen, sind verschiedene Vorgaben zu beachten. Zunächst soll das fluidabgebende Kissen eine ausreichende Menge an Fluid aufnehmen können, um auch eine Benutzung über einen längeren Zeitraum zu ermöglichen. Dabei soll auch vermieden werden, dass das Fluid unkontrolliert austritt oder verdunstet und möglichst nur bei der Benutzung des fluidabgebenden Kissens freigesetzt wird. Zu diesem Zweck ist im Rahmen der Erfindung die elastische, perforierte Folie vorgesehen, wobei die Perforation vorzugsweise durch Einschnitte gebildet ist, welche eine gewisse Ventilfunktion ermöglichen. Im unverformten Zustand der elastischen Folie sind die einzelnen Poren der Perforation nur klein oder vorzugsweise durch die elastische Rückstellung geschlossen. Wenn jedoch die elastische Folie als Frontwand des fluidabgebenden Kissens bei der Benutzung durch den Druck eines Benutzers verformt wird, können sich die Poren lokal öffnen, wodurch dann Fluid abgegeben wird.

Da die Aufnahmemenge des fluidabgebenden Kissens begrenzt ist und über einen längeren Zeitraum auch Verschmutzungen nicht ausgeschlossen werden können, soll das fluidabgebende Kissen auch leicht zu erneuern sein, wobei dann ein solches Wegwerfprodukt kostengünstig und leicht herstellbar sein muss.

Des Weiteren bildet die Frontwand als Kontaktfläche die sichtbare Oberseite des fluidabgebenden Kissens. In vielen Fällen soll einem Benutzer angezeigt werden, dass nur genau das fluidabgebende Kissen als Kontaktfläche genutzt werden soll. Die Frontwand ist deshalb in der Regel bedruckt, wobei der Aufdruck sowohl dauerhaft als auch hochwertig sein soll und auch zu Werbezwecken genutzt werden kann.
WO03028456 offenbart einen imprägnierten Pad, das von einer dichten Schale teils umgeben ist, welche mit einer Folie verschweißt ist, welche durch Coextrusion hergestellt sein kann.

Fluidabgebende Kissen mit den eingangs beschriebenen Merkmalen sind aus WO 2013/167746 A2 und US 2011/0111000 A1 bekannt. Ähnliche mit einem Desinfektionsmaterial versehene Kissen werden des Weiteren in US 4 832 942, US 7 037 569 B2, US 7 722 589 B2 und EP 2 098 664 A1 beschrieben.

Bei den bekannten Ausgestaltungen ist die Bedruckbarkeit der Frontwand verbesserungsbedürftig .

Vor diesem Hintergrund liegt der vorliegenden Erfindung die Aufgabe zugrunde, die Verwendung einer verbesserten elastischen perforierten Folie anzugeben.

Gegenstand der Erfindung und Lösung der Aufgabe ist eine Verwendung gemäß Patentanspruch 1.

Ausgehend von dem Oberbegriff des Patentanspruches 1 ist erfindungsgemäß vorgesehen, dass die elastische Folie eine zumindest zweischichtige Coextrusionsfolie mit einer elastischen Schicht und einer nicht elastischen, als eine Oberfläche des fluidabgebenden Kissens vorgesehenen Deckschicht aus Polyolefin ist, wobei die Dicke der Deckschicht weniger als 15 µm beträgt und wobei das Dickenverhältnis der elastischen Schicht zu der Deckschicht zumindest 10:1 ist.

Die Deckschicht aus Polyolefin kann leicht bedruckt werden, wozu vorzugsweise auch eine Corona-Behandlung der Deckschicht erfolgt. Durch Coextrusion kann die Deckschicht jedoch so dünn ausgeführt werden, dass die von der elastischen Schicht bereitgestellten elastischen Eigenschaften der perforierten elastischen Folie nicht wesentlich beeinträchtigt werden. Insbesondere ist die Deckschicht nur auf der Seite vorgesehen, welche die Kontaktfläche und damit die Oberfläche des fluidabgebenden Kissens bildet. Die gegenüberliegende, mit der Rückwand verbundene Seite ist dagegen aus einem elastischen Material bei einer bevorzugt genau zweischichtigen Ausgestaltung der Coextrusionsfolie von der elastischen Schicht gebildet.

Das Dickenverhältnis der elastischen Schicht zu der Deckschicht beträgt zumindest 10:1. Auch durch diese starke Asymmetrie wird erreicht, dass die elastische Folie gute elastische Eigenschaften und eine ausreichende Beweglichkeit aufweist, wobei die dünne Deckschicht diese Eigenschaften nicht wesentlich beeinträchtigt, jedoch eine gute Bedruckbarkeit gewährleistet.

Selbst wenn die dünne nicht elastische Schicht eine gewisse Versteifung bewirkt, können die durch die Perforation gebildeten Poren oder Schlitze zumindest durch die Asymmetrie der Coextrusionsfolie an der nach innen in Richtung des Kerns gewandten Seite geschlossen werden. Eine leichte Versteifung an der zu bedruckenden Deckschicht ist hinsichtlich der Verarbeitung der elastischen Folie sogar von Vorteil, weil die elastische Folie sich bei ihrer Verarbeitung und insbesondere dem Bedrucken weniger dehnt. Der vorliegenden Erfindung liegt in diesem Zusammenhang die Erkenntnis zugrunde, dass mit der elastischen Schicht nicht wie üblich eine gute elastische Dehnbarkeit der gesamten Frontwand, sondern vielmehr ein lokaler Verschluss der durch die Perforation gebildeten Poren bezweckt wird.

Die elastische Folie ist vorzugsweise ausgehend von einer Ausgangslänge um zumindest 50 % dehnbar, wobei dann bei einem Wegfall der für die Dehnung aufgebrachten Kraft die bleibende Verformung im Vergleich zu der Ausgangslänge typischerweise weniger als 20 %, vorzugsweise weniger als 10 % beträgt.

Die aus Polyolefin gebildete und bevorzugt mit einer Corona-Entladung behandelte Deckschicht ist im Rahmen der Erfindung üblicherweise bedruckt bzw. bedruckbar. Als Material für die Deckschicht kommen insbesondere Polyethylen und Polypropylen in Betracht, wobei eine Mischung von Polypropylen (PP) und Polyethylen (PE) besonders bevorzugt ist. Beispielsweise kann der Anteil von Polyethylen in der Deckschicht zwischen 40 und 70 Gew.-% und der Anteil von Polypropylen zwischen 30 und 60 Gew.-% liegen, wobei als weitere Bestandteile mit einem Anteil von vorzugsweise weniger als 10 Gew.-% Talkum, Verarbeitungshilfsmittel oder weitere Zusatzstoffe vorgesehen sein können.

Bevorzugte Polyolefine für die Deckschicht sind lineares Polyethylen niedriger Dichte (LLDPE) und Polypropylen-Copolymer.

Gemäß einer besonders bevorzugten Ausgestaltung der Erfindung enthält die Deckschicht auch einen Absorber für niedermolekulare Bestandteile, wie beispielsweise Wasser und Stryrol.

Da die elastische Folie durch Coextrusion gebildet ist, kann die Deckschicht sehr dünn ausgeführt werden, wobei abhängig von der Materialzusammenstellung bei der Coextrusion auch keine weiteren Haftvermittlerschichten oder dergleichen notwendig sind. Bevorzugt beträgt die Dicke der Deckschicht weniger als 10 µm und mindestens 2 µm, beispielsweise 4 µm.

Die elastische Schicht enthält vorzugsweise zwischen 20 und 70 Gew.-% Styrol-Block-Copolymer (SBC) und zwischen 15 und 50 Gew.-% Ethylen-Vinylacetat (EVA). Als Styrol-Block-Copolymer kommen beispielsweise StyrolButadien-Styrol-Copolymer (SBS), Styrol-Ethenbuten-Styrol-Copolymer (SEBS), Styrol-Ethenpropen-Styrol-Copolymer (SEPS) und Styrol-Isopren-Styrol-Copolymer (SIS) in Betracht, wobei SBS bevorzugt ist.

Ethylen-Vinylacetat (EVA) ist insbesondere zweckmäßig, um eine gute Siegelbarkeit gegenüber der Rückwand zu erreichen, wenn diese gemäß einer bevorzugten Ausgestaltung der Erfindung aus Polyethylenterephthalat (PET) gebildet ist. Zu diesem Zweck liegt auch der Vinylacetat-Gehalt in dem Ethylen-Vinylacetat über 10 % und bevorzugt über 15 %. Der Vinylacetat-Gehalt in dem Ethylen-Vinylacetat kann beispielsweise 10 % bis 30 %, vorzugsweise zwischen 15 % und 25 % betragen.

Die Rückwand weist üblicherweise eine Schalenform auf, wobei die Rückwand insbesondere von einer tiefgezogenen Schale gebildet sein kann, wobei neben PET beispielsweise auch Polystryrol (PS) und Polystyrol-Copolymer als Material in Betracht kommen. Die von der elastischen Folie gebildete Frontwand ist dann vorzugsweise mit einem umlaufenden, abgewinkelten Rand der Schale versiegelt und deckt die von der Schale gebildete Mulde mit dem darin angeordneten Kern ab. Im Rahmen einer solchen Ausgestaltung kann die elastische Folie im Wesentlichen eben oder lediglich leicht gewölbt sein, so dass diese dann im unbelasteten Zustand weitgehend kräftefrei angeordnet ist, wodurch aufgrund der elastischen Eigenschaften die Perforation mit einer Art Ventilfunktion auch an den Rändern geschlossen sein kann. Grundsätzlich ist es auch möglich, die Perforation nur in einem Teilbereich der Frontwand anzuordnen, wobei beispielsweise ein unterer Rand oder ein umlaufender Rand von der Perforation ausgespart sein können.

Vorzugsweise ist die elastische Folie durch Einschnitte perforiert, wobei die Einschnitte eine Ventilfunktion aufweisen und im unverformten Zustand der elastischen Folie zumindest weitgehend oder vorzugsweise vollständig geschlossen sind.

Hinsichtlich der konkreten Ausgestaltung des fluidabgebenden Kissens ergeben sich im Rahmen der Erfindung verschiedene Möglichkeiten. Die Rückwand kann wie zuvor beschrieben als steife, tiefgezogene Schale ausgeführt sein, wobei dann die Rückwand direkt oder über einen separaten Träger auf der Kontaktfläche einer Tür, eines Schalters oder dergleichen angeordnet werden kann. Es sind jedoch auch Ausgestaltungen denkbar, bei denen die Rückwand flexibel ist, um das fluidabgebende Kissen auf eine gekrümmte Oberfläche, beispielsweise eine Griffstange oder dergleichen aufbringen zu können.

Die Dicke der elastischen Schicht liegt typischerweise zwischen 80 µm und 300 µm, vorzugsweise zwischen 100 µm und 200 µm, was aufgrund der lediglich geringen Dicke der Deckschicht auch in etwa der Gesamtdicke der elastischen Folie entspricht.

Wie bereits eingangs erläutert, ist das Fluid des fluidabgebenden Kissens vorzugsweise eine desinfizierende Flüssigkeit oder ein desinfizierendes Gel.

Um das Fluid halten zu können, weist der Kern zweckmäßigerweise ein geeignetes Trägermaterial auf, wobei es sich beispielsweise um ein Textil, ein Nonwoven, Watte oder Schaumstoff handeln kann. Einerseits muss dieses Material Hohl- und Freiräume für das Fluid aufweisen. Andererseits sind aber auch gewisse saugende Eigenschaften von Vorteil, um das Fluid in einem gewissen Maße halten zu können, damit dieses nur bei einem Druck auf die elastische, perforierte Folie abgegeben wird. Darüber hinaus ist das Trägermaterial bevorzugt in einem gewissen Maße auch druckelastisch.

Gegenstand der Erfindung ist schließlich auch eine Desinfektionseinrichtung mit dem fluidabgebenden Kissen, dessen Rückwand von einer tiefgezogenen Schale gebildet ist, wobei als Fluid die desinfizierende Flüssigkeit oder das desinfizierende Gel vorgesehen ist. Das fluidabgebende Kissen ist auswechselbar von einem Träger gehalten. Ein solcher Träger kann von einer tiefgezogenen Schale gebildet sein, welche dann dauerhaft auf einer Kontaktfläche befestigt wird, während das fluidabgebende Kissen selbst leicht ausgewechselt werden kann. Der Träger kann beispielsweise eine tiefgezogene Schale aus Polyethylenterephthalat sein.

Wenn sowohl der Träger als auch die Rückwand des fluidabgebenden Kissens als tiefgezogene Schale gebildet sind, kann die Rückwand auch auf eine einfache Weise durch eine Art Rastverbindung lösbar in dem Träger gehalten sein.

Die Erfindung wird im Folgenden anhand einer lediglich ein Ausführungsbeispiel darstellenden Zeichnung erläutert. Es zeigen:
- Fig. 1: eine Desinfektionseinrichtung mit einem fluidabgebenden Kissen und einem Träger,
- Fig. 2: das fluidabgebende Kissen gemäß der Fig. 1 in einer Schnittdarstellung,
- Fig. 3: einen Schnitt durch eine Frontwand des in Fig. 2 dargestellten fluidabgebenden Kissens,
- Fig. 4: eine Schnittdarstellung entsprechend der Fig. 2 mit dem fluidabgebenden Kissen und dem Träger gemäß der Fig. 1.

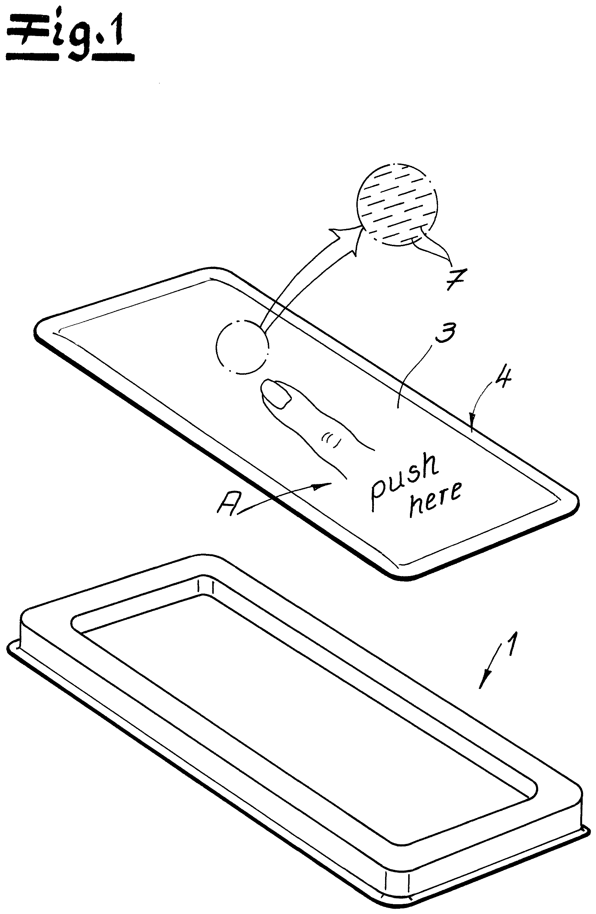

Die Fig. 1 zeigt ein fluidabgebendes Kissen sowie einen Träger 1 für das fluidabgebende Kissen. Der Träger 1 kann auf eine Kontaktfläche einer Tür oder dergleichen aufgebracht werden, wobei dann das fluidabdeckende Kissen auswechselbar an dem Träger 1 durch eine Rastverbindung gehalten ist.

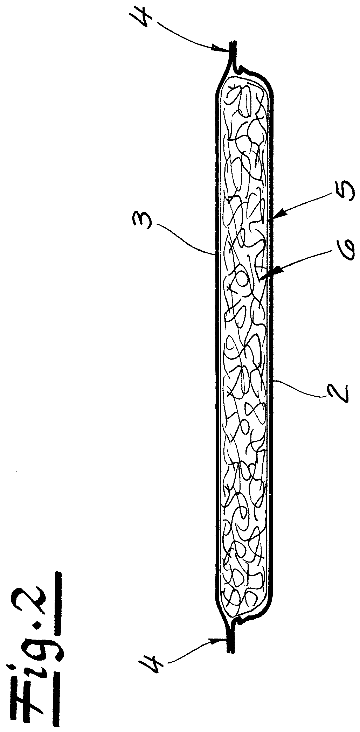

Die Details des fluidabgebenden Kissens sind in der Schnittdarstellung gemäß der Fig. 2 ersichtlich. Das fluidabgebende Kissen weist eine fluiddichte Rückwand 2 auf, die die Form einer Schale aufweist und aus einer PET-Folie tiefgezogen ist. An der gegenüberliegenden Vorderseite des fluidabgebenden Kissens ist eine perforierte elastische Folie 3 angeordnet, welche durch eine umlaufende Heißsiegelnaht 4 mit der Rückwand 2 verbunden ist. Zwischen der elastischen Folie 3 als Frontwand und der Rückwand 2 ist ein fluidaufnehmender Kern 5 angeordnet, wobei der fluidaufnehmende Kern ein Trägermaterial 6 ausgewählt aus der Gruppe Textil, Nonwoven, Watte und Schaumstoff aufweist und wobei in dem Kern 5 das Fluid in Form einer desinfizierenden Flüssigkeit oder eines desinfizierenden Gels aufgenommen ist. Das Trägermaterial 6 ist vorzugsweise auch druckelastisch, so dass sich in einem kräftefreien Zustand stets die Form des fluidabgegebenen Kissens gemäß der Fig. 2 ergibt.

Gemäß der Fig. 1 ist die Perforation durch eine Vielzahl von Einschnitten 7 in der elastischen Folie 3 gebildet. Dadurch ist es möglich, dass die Einschnitte 7 aufgrund der elastischen Eigenschaften der elastischen Folie 3 eine Ventilfunktion aufweisen, wobei die Einschnitte 7 im unverformten Zustand der elastischen Folie 3 zumindest weitgehend geschlossen sind, so dass das Fluid in dem fluidaufnehmenden Kern 5 zurückgehalten wird.

Bei der Verformung der elastischen Folie 3 durch die Berührung eines Benutzers kann sich die elastische Folie 3 dann derart verformen, dass an den einzelnen Einschnitten Poren geöffnet werden und Fluid abgegeben wird.

In der Fig. 1 ist des Weiteren angedeutet, dass die elastische Folie 3 an der freiliegenden Oberfläche mit einem Aufdruck A versehen ist. Einerseits soll das fluidabgebende Kissen ein hochwertiges Erscheinungsbild aufweisen und andererseits muss einem Benutzer auch angezeigt werden, dass das fluidabgebende Kissen als Teil einer Desinfektionseinrichtung als Kontaktfläche genutzt werden soll.

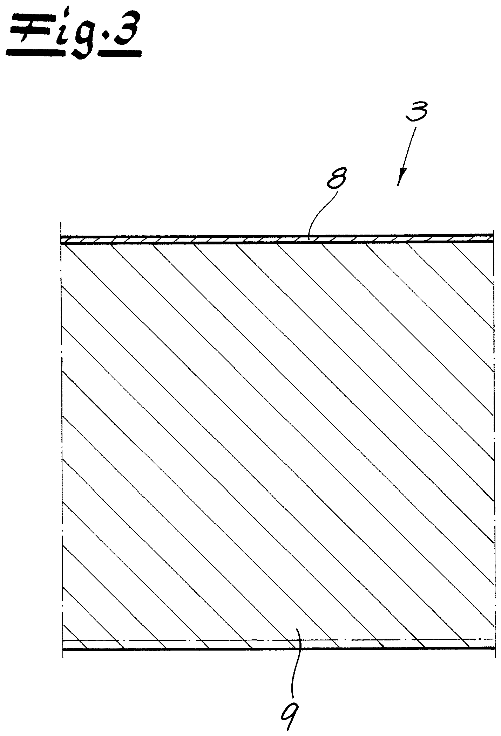

Wie in der Fig. 3 dargestellt, ist die elastische Folie 3 erfindungsgemäß von einer zumindest zweischichtigen Coextrusionsfolie gebildet, welche eine nicht elastische, eine Oberfläche des fluidabgebenden Kissens bildende dünne Deckschicht 8 aus Polyolefin und eine elastische Schicht 9 aufweist. Die elastische Schicht 9 ist in Richtung des fluidaufnehmenden Kerns 5 angeordnet, wobei die gegenüberliegende dünne Deckschicht erfindungsgemäß eine Dicke von weniger als 10 µm aufweist. Das Dickenverhältnis der elastischen Schicht 9 zu der Deckschicht 8 beträgt zumindest 10:1.

In dem konkret dargestellten Ausführungsbeispiel weist die Außenschicht eine Dicke von 4 µm auf und enthält 55 Gew.-% LLDPE, 40 Gew.-% PP-Copolymer und Rest Talkum, Verarbeitungshilfsmittel und Absorber. Der Absorber ist speziell dazu vorgesehen, um niedermolekulare Bestandteile aufzunehmen, welche die elastische Schicht 9 verlassen können.

Die Deckschicht ist mit einer Corona-Entladung behandelt, um die Bedruckbarkeit zu verbessern.

Die Kernschicht weist insgesamt eine Dicke von 196 µm auf, so dass sich in dem konkreten Ausführungsbeispiel ein Dickenverhältnis von 49:1 ergibt.

Die elastische Schicht besteht zu 40 Gew.-% aus SBS und 35 Gew.-% EVA mit einem Vinylacetatgehalt von 18 %. Des Weiteren enthält die elastische Schicht Verarbeitungshilfsmittel, Farbpigmente, Gleitmittel, Antiblockmittel und Stabilisatoren.

Es resultiert also eine stark asymmetrische zweischichtige Coextrusionsfolie.

In dem Ausführungsbeispiel gemäß der Fig. 3 ist diese an sich zweischichtige Coextrusionsfolie mit einer für drei Schichten eingerichteten Extrusionsanlage hergestellt. Zu diesem Zweck wird die elastische Schicht 9 aus zwei Teilschichten mit gleicher Materialzusammensetzung gebildet, wobei die in dem Schichtaufbau mittlere Teilschicht eine Dicke von 192 µm und die weitere Teilschicht eine Dicke von 4 µm aufweist. Der Schichtübergang ist aber bei der Coextrusionsfolie in der Regel nicht mehr erkennbar, weil die Teilschichten bei der Coextrusion miteinander verschmelzen. Der Schichtübergang ist in der Fig. 3 durch eine strichpunktierte Linie angedeutet.

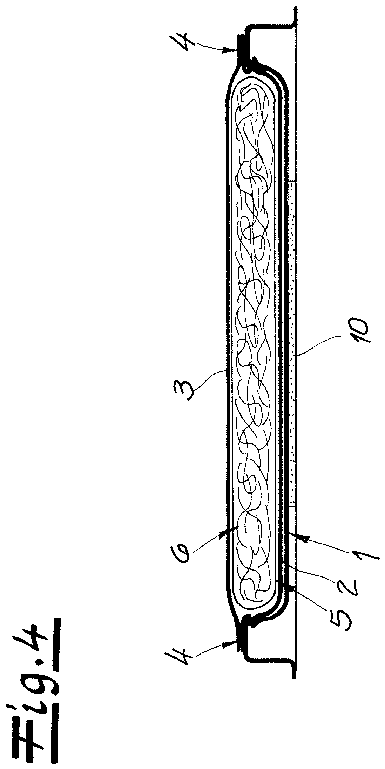

Die Fig. 4 zeigt schließlich das fluidabgebende Kissen, welches auf dem Träger 1 angeordnet ist und beispielsweise durch eine Rastverbindung gehalten werden kann, damit das fluidabgebende Kissen leicht ausgewechselt werden kann. Der Träger 1 kann beispielsweise mit Klebestreifen 10 auf einer Kontaktfläche einer Tür oder dergleichen befestigt sein.

## Patentansprüche

1. Verwendung einer perforierten elastischen Folie (3) als Frontwand eines fluidabgebenden Kissens, welches eine fluiddichte Rückwand (2) aus Kunststoff, und einen fluidaufnehmenden Kern (5) zwischen der Frontwand und der Rückwand (2) aufweist, wobei die perforierte elastische Folie (3) durch eine Heißsiegelnaht (4) mit der Rückwand (2) verbunden wird, **dadurch gekennzeichnet, dass** die elastische Folie (3) eine zumindest zweischichtige Coextrusionsfolie mit einer elastischen Schicht (9) und einer nicht elastischen, als eine Oberfläche des fluidabgebenden Kissens vorgesehenen Deckschicht (8) aus Polyolefin ist, wobei die Dicke der Deckschicht (8) weniger als 15 µm beträgt und wobei das Dickenverhältnis der elastischen Schicht (9) zu der Deckschicht (8) zumindest 10:1 ist.

2. Verwendung nach Anspruch 1, **dadurch gekennzeichnet, dass** die elastische Folie (3) durch Einschnitte (7) perforiert ist, wobei die Einschnitte (7) eine Ventilfunktion bewirken und im unverformten Zustand der elastischen Folie (3) geschlossen sind.

3. Verwendung nach Anspruch 1 oder 2, **dadurch gekennzeichnet, dass** die Deckschicht (8) bedruckt ist.

4. Verwendung nach einem der Ansprüche 1 bis 3, **dadurch gekennzeichnet**, die perforierte elastische Folie (3) mit der Rückwand (2) aus Polyethylenterephthalat (PET) verbunden wird.

5. Verwendung nach einem der Ansprüche 1 bis 4, **dadurch gekennzeichnet, dass** die perforierte elastische Folie (3) mit einer tiefgezogenen Schale als Rückwand (2) verbunden wird, wobei die perforierte elastische Folie (3) mit einem umlaufenden abgewinkelten Rand der Schale versiegelt und dadurch in einer Ebene gehalten wird.

6. Verwendung nach einem der Ansprüche 1 bis 5, **dadurch gekennzeichnet, dass** die elastische Schicht (9) zwischen 20 Gew.-% und 70 Gew.-% Styrol-Block-Copolymer (SBC) und zwischen 15 Gew.-% und 50 Gew.-% Ethylen-Vinylacetat (EVA) enthält.

7. Verwendung nach Anspruch 6, **dadurch gekennzeichnet, dass** der Vinylacetat-Gehalt in dem Ethylen-Vinylacetat zwischen 10 % und 30 %, vorzugsweise zwischen 15 % und 25 % beträgt.

8. Verwendung nach einem der Ansprüche 1 bis 7, **dadurch gekennzeichnet, dass** die elastische Schicht (9) der elastischen Folie (3) mit der Rückwand (2) versiegelt wird.

9. Verwendung nach einem der Ansprüche 1 bis 8, **dadurch gekennzeichnet, dass** die elastische Schicht (9) eine Dicke zwischen 80 µm und 300 µm aufweist.

10. Verwendung nach einem der Ansprüche 1 bis 9, **dadurch gekennzeichnet, dass** die Deckschicht (8) ein Gemisch aus Polypropylen (PP) und Polyethylen (PE) ist.

11. Verwendung nach Ansprüche 10, **dadurch gekennzeichnet, dass** die Deckschicht (8) ein lineares Polyethylen niedriger Dichte (LLDPE) und Polypropylen-Copolymer ist.

## Claims

1. Use of a perforated elastic film (3) as front wall of a fluid-dispensing pad which comprises a fluid-tight rear wall (2) made of plastic and a fluid-retaining core (5) between the front wall and the rear wall (2), wherein the perforated elastic film (3) is connected by a heat-seal seam (4) to the rear wall (2), **characterized in that** the elastic film (3) is at least a two-layer co-extruded film having an elastic layer (9) and a non-elastic cover layer (8) made of polyolefin, provided as a surface of the fluid-dispensing pad, wherein the thickness of the cover layer (9) is less than 15 µm and wherein the thickness ratio of the elastic layer (9) to the cover layer (8) is at least 10:1.

2. The use according to claim 1, **characterized in that** the elastic film (3) is perforated by incisions (7) wherein the incisions (7) bring about a valve effect and are closed in the undeformed state of the elastic film (3).

3. The use according to claim 1 or 2, **characterized in that** the cover layer (8) is printed.

4. The use according to any one of claims 1 to 3, **characterized in that** the perforated elastic film (3) is connected to the rear wall (2) made of polyethylene terephthalate (PET).

5. The use according to any one of claims 1 to 4, **characterized in that** the perforated elastic film (3) is connected to a deep-drawn shell as rear wall (2), wherein the perforated elastic film (3) is sealed with a circumferential bent edge of the shell and thereby held in a plane.

6. The use according to any one of claims 1 to 5, **characterized in that** the elastic layer (9) contains between 20 wt.% and 70 wt.% of styrene block copolymer (SBC) and between 15 wt.% and 50 wt.% of ethylene vinyl acetate (EVA).

7. The use according to claim 6, **characterized in that** the vinyl acetate content in the ethylene vinyl acetate is between 10 % and 30 %, preferably between 15% and 25%.

8. The use according to any one of claims 1 to 7, **characterized in that** the elastic layer (9) of the elastic film (3) is sealed with the rear wall (2).

9. The use according to any one of claims 1 to 8, **characterized in that** the elastic layer (3) has a thickness between 80 µm and 300 µm.

10. The use according to any one of claims 1 to 9, **characterized in that** the cover layer (8) is a mixture of polypropylene (PP) and polyethylene (PE).

11. The use according to claim 10, **characterized in that** the cover layer (8) is a linear low density polyethylene (LLDPE) and a polypropylene copolymer (PP).

## Revendications

1. Emploi d'un film élastique perforé (3) en tant que paroi avant d'un coussinet libérant un fluide, lequel présente une paroi arrière (2) en plastique étanche aux fluides et un coeur (5) absorbant les fluides entre la paroi avant et la paroi arrière (2), dans lequel le film élastique perforé (3) est relié à la paroi arrière (2) par une thermosoudure (4), **caractérisé en ce que** le film élastique (3) est un film coextrudé au moins bi-couches avec une couche élastique (9) et une couche de finition (8) en polyoléfine, non élastique et prévue en tant qu'une surface du coussinet libérant du fluide, dans lequel l'épaisseur de la couche de finition (8) est inférieure à 15 µm et dans lequel l'épaisseur relative de la couche élastique (9) à la couche de finition (8) est au moins de 10:1.

2. Emploi selon la revendication 1, **caractérisé en ce que** le film élastique (3) est perforé par des encoches (7), dans lequel les encoches (7) ont une fonction d'aération et sont fermées lorsque le film élastique (3) est dans l'état non déformé.

3. Emploi selon la revendication 1 ou 2, **caractérisé en ce que** la couche de finition (8) est imprimée.

4. Emploi selon l'une des revendications 1 à 3, **caractérisé en ce que** le film élastique perforé (3) est relié à la paroi arrière (2) en polyéthylène téréphtalate (PET).

5. Emploi selon l'une des revendications 1 à 4, **caractérisé en ce que** le film élastique perforé (3) est relié à une coque emboutie en tant que paroi arrière (2), dans lequel le film élastique perforé (3) est scellé avec un bord périphérique coudé de la coque et est maintenu ainsi dans un plan.

6. Emploi selon l'une des revendications 1 à 5, **caractérisé en ce que** la couche élastique (9) contient entre 20 % en poids et 70 % en poids de copolymère styrène-butadiène (SBC) et entre 15 % en poids et 50 % en poids d'éthylène-acétate de vinyle (EVA).

7. Emploi selon la revendication 6, **caractérisé en ce que** la teneur en acétate de vinyle dans l'éthylène-acétate de vinyle est entre 10 % et 30 %, de préférence entre 15 % et 25 %.

8. Emploi selon l'une des revendications 1 à 7, **caractérisé en ce que** la couche élastique (9) du film élastique (3) est scellée à la paroi arrière (2).

9. Emploi selon l'une des revendications 1 à 8, **caractérisé en ce que** la couche élastique (9) présente une épaisseur entre 80 µm et 300 µm.

10. Emploi selon l'une des revendications 1 à 9, **caractérisée en ce que** la couche de finition (8) est un mélange de polypropylène (PP) et de polyéthylène (PE).

11. Emploi selon la revendication 10, **caractérisée en ce que** la couche de finition (8) est un polyéthylène basse densité linéaire (LLDPE) et un copolymère de polypropylène (PP).
